Europäisches Patentamt

European Patent Office

Office européen des brevets

(19)

(11) Numéro de publication: **0 397 583**

**A1**

## (12) DEMANDE DE BREVET EUROPEEN

(21) Numéro de dépôt: 90420212.4

(22) Date de dépôt: 02.05.90

(51) Int. Cl.5: **C12Q 1/04, G01N 33/50,
G01N 33/04, G01N 1/30**

(30) Priorité: 12.05.89 FR 8906525

(43) Date de publication de la demande:
14.11.90 Bulletin 90/46

(84) Etats contractants désignés:
BE DE FR GB NL

(71) Demandeur: **L.E.C.A. ( LABORATOIRE
D'ETUDES EN CYTOMETRIE APPLIQUEE)
20 cours Becquart Castelbon
F-38500 Voiron(FR)**

(72) Inventeur: **Batandier, Cécile
4 rue Charles Testoud
F-38000 Grenoble(FR)**
Inventeur: **Perrot, Guy
La Murette
F-38140 Rives(FR)**

(74) Mandataire: **Laurent, Michel et al
Cabinet Laurent et Charras 20, rue Louis
Chirpaz B.P. 32
F-69131 Ecully Cédex(FR)**

(54) **Procédé et dispositif d'identification et de numérisation de cellules dans du lait.**

(57) Procédé pour identifier et numériser les cellules contenues dans du lait **caractérisé** en ce qu'il consiste:

- à prélever une quantité déterminée d'un échantillon d'un lait à analyser ;

- à colorer les cellules présentes dans cet échantillon par un colorant fluorescent spécifique de l'ADN contenu dans le noyau desdites cellules ;

- à faire défiler cet échantillon coloré dans un tube capillaire (13) sur lequel est dirigé un faisceau lumineux stabilisé ;

- à recueillir sur un capteur une partie au moins du rayon lumineux transmis, donnant une première information, représentative du nombre de cellules qui défilent ;

- à traiter cette information ;

- et enfin, à restituer au moins une valeur représentative de la qualité du lait analysé.

Application : diagnostic de la mammite chronique chez les vaches.

## PROCEDE ET DISPOSITIF D'IDENTIFICATION ET DE NUMERISATION DE CELLULES DANS DU LAIT.

La présente invention concerne un procédé pour détecter et numériser les cellules présentes dans du lait et notamment dans du lait de vache. Elle concerne également le dispositif mettant en oeuvre ce procédé.

Les produits alimentaires d'origine animale sont soumis à un contrôle vétérinaire qui, au fur et à mesure des progrès de la recherche scientifique, tendent à s'intensifier, afin de proposer au consommateur des produits de qualité.

Les produits laitiers sont particulièrement soumis à ces contrôles, du fait de leur propension à rapidement s'altérer. Parmi ceux-ci, le lait de vache, très largement consommé, subit des examens vétérinaires systématiques, notamment en vue de révéler la présence en nombre relativement important de cellules, d'origine épithéliale en phase de désquamation et en voie de lyse, mais également d'origine inflammatoire, tels que des lymphocytes et autres leucocytes, révélatrices de pathologie de la vache concernée, et notamment de mammites chroniques.

Cette infection, bien que sans grande incidence sur l'état général de la vache, rend le lait tout à fait impropre à la consommation. De fait, un dépistage systématique de cette infection est réalisé sur le lait. Ce dépistage peut être effectué à différents niveaux, soit par le producteur dès que la traite est effectuée, soit par des services vétérinaires, soit par les laiteries ou les industries de transformation.

A ce jour, on utilise deux types de techniques . La première consiste en une appréciation approximative du nombre de cellules contenues dans un échantillon, à l'aide d'un détergent, du type connu sous la marque déposée TEEPOL, ou d'un indicateur colorimétrique. La deuxième technique, utilisée par les professionnels, consiste en un comptage des cellules soit de façon manuelle,impliquant la présence de personnel spécialisé, soit de façon automatisée au moyen d'un matériel approprié, qui ne peut hélas différencier les cellules entières des débris cellulaires, et qui de plus demande un étalonnage manuel préalable sur au moins deux échantillons.

Parmi ces différentes méthodes, HAGELTORN et al ont proposé (voir BIOLOGICAL ABSTRACTS, vol. 82, n° 12,1986, page AB-458, & AM. J. VET.RES., 47(9), pages 2012-2016, 1986) un procédé apte à identifier des cellules contenant dans de l'ADN ou acide désoxyribonucléïque, que de l'ARN ou acide ribonucléïque, marquées préalablement de manière spécifique au moyen d'un colorant supravital, l'Acridine Orange. Cette identification est réalisée grâce à une localisation géographique des différentes populations cellulaires repertoriées sur un diagramme de répartition, obtenu après analyse en cytométrie de la taille et de la densité desdites cellules. Ce procédé vise plus spécifiquement à déterminer les excès de ces diverses populations cellulaires par rapport à des données standard, répertoriées dans des banques de données, qu'à réaliser un comptage cellulaire des échantillons étudiés. Ces excès obtenus de manière relative, ne permettent pas d'aboutir à des valeurs absolues, qui seules sont réellement représentatives de la qualité du lait analysé. En outre, pour le colorant spécifique utilisé, la coloration stoechiométriques sur l'ADN et sur l'ARN n'est obtenue que pour des conditions opératoires strictes, donc incompatibles avec une technique automatisée. Il est donc inutilisable dans le cadre d'analyse pour lesquelles la totalité des cellules, tant mortes que vivantes doivent être prises en compte, et notamment, dans le cas du dépistage systématique des mammites chroniques.Enfin, comme déjà dit, ce procédé reposant sur la comparaison avec des données de base, préalablement intégrées, suppose une connaissance approfondie du domaine considéré, et demande donc un personnel qualifié pour sa mise en oeuvre.

On a également proposé dans le brevet US-A-3679365 une méthode de comptage des cellules somatiques dans le lait, consistant à colorer préalablement la totalité de l'échantillon de lait à analyser. De la sorte, et afin de pouvoir réaliser le comptage ultérieur, une étape supplémentaire dite de "clarification", destinée à faire éclater les globules lipidiques au moyen d'un agent tensio-actif est rendue nécessaire, accroissant de fait la durée des analyses .

On a également proposé une méthode plus spécifiquement destinée à l'identification de bactéries, et reposant sur le principe de la fluorométrie. Cette méthode, décrite par exemple par SHELLY et al dans CLINICAL CHEMISTRY, vol.29, n° 2, 1983, pages 290-296, consiste à faire subir à un échantillon une double coloration, à l'origine d'une double fluorescence, dont le rapport relatif mesuré et analysé permet de remonter au type de bactéries. Ce procédé suppose donc la connaissance préalable des rapports relatifs de fluorescence de chaque type de bactérie, et donc corollairement, ne peut être employé que par du personnel qualifié. En outre, si certes, l'identification des bactéries est obtenue de manière relativement précise, en revanche, cette méthode s'avère impropre pour la numérisation cellulaire, et donc pour le dépistage des mammites chroniques, objet principal de l'invention.

La présente invention vise à pallier ces inconvénients. Elle vise un procédé et un dispositif apte à permettre un diagnostic infraclinique de la mammite chronique, qui soit sûr et sans appel, par voie automatique, précis, rapide et de coût de fonctionnement léger.

Ce procédé pour identifier et numériser les cellules dans du lait est caractérisé en ce qu'il consiste:

- à prélever un échantillon d'un lait à analyser ;
- à colorer les cellules présentes dans cet échantillon par un colorant fluorescent spécifique de l'ADN (Acide desoxyribonucléïque) contenu dans le noyau des cellules ;
- à faire défiler cet échantillon coloré dans un tube capillaire sur lequel est dirigé un faisceau laser stabilisé ;
- à recueillir sur un capteur une partie au moins du rayon lumineux transmis à travers le flux de l'échantillon s'écoulant dans le tube capillaire pour donner une première information $I_1$, significative de la quantité d'ADN, donc du nombre de cellules qui défilent ;
- à transmettre ladite information $I_1$ à une unité de traitement ;
- et enfin, à restituer au moins une valeur représentative de la qualité du lait analysé.

En d'autres termes, la présente invention consiste à analyser par cytofluorométrie un échantillon de lait préalablement coloré par un colorant approprié, afin de déterminer notamment la quantité et la qualité des cellules contenues dans ledit échantillon de lait, et à traiter les informations ainsi recueillies pour déterminer la qualité de lait contenue dans ledit échantillon.

Avantageusement, en pratique :

- lors de la phase de coloration, on ajoute en outre un autre colorant fluorescent, apte à induire une réaction enzymatique spécifique d'une famille des constituants contenus dans lesdites cellules, puis on recueille sur un capteur, partie du rayon lumineux transmis à travers le flux de l'échantillon s'écoulant dans le tube capillaire pour donner une information, représentative de la quantité de la dite famille de constituants cellulaire ;
- simultanément, on recueille sur des capteurs, respectivement :
. une partie du rayon lumineux diffracté à 90° de l'axe optique défini par le faisceau lumineux initial pour donner une seconde information $I_2$, significative de la densité desdites cellules ;
. au moins une partie du rayon lumineux légèrement diffracté au voisinage de l'axe optique, pour donner une troisième information $I_3$, significative de la taille desdites cellules ;
les dites informations $I_2$ et $I_3$ étant alors transmises à la dite unité de traitement puis restituées sous forme de valeur(s) représentative(s) de la qualité du lait analysé ;

- simultanément à la coloration, on ajoute au lait un milieu hypotonique, destiné à lyser la membrane cellulaire, et un détergent tensio-actif destiné à perméabiliser la membrane nucléaire afin de permettre la pénétration du colorant fluorescent destiné à venir se fixer sur l'ADN ;
- l'agent tensio-actif est introduit dans le milieu à raison de 0,1 % en volume, et l'agent hypotonique est du citrate de sodium à raison de 0,1 % en rapport Poids/Volume ;
- le colorant fluorescent spécifique de l'ADN est de l'iodure de propidium, introduit en excès par rapport à la quantité d'ADN ;
- on laisse incuber le milieu réactionnel à température ambiante et dans l'obscurité pendant au moins vingt minutes ;
- on fait défiler le milieu réactionnel au moyen d'un venturi à pression différentielle.

L'invention concerne également un dispositif pour la mise en oeuvre du procédé comprenant :
- un rayon lumineux stabilisé ;
- une unité d'analyse constituée par :
. une cellule de lecture réalisée en quartz, située sur le trajet optique du faisceau laser ;
. un système à pression différentielle destiné à permettre le passage dans ladite cellule de lecture de l'échantillon de lait à analyser ;
. un réservoir destiné à recueillir l'échantillon après analyse ;
- au moins trois capteurs optiques dont deux sont munis d'un photomultiplicateur ;
- un microprocesseur apte à assurer le traitement des informations issues dudit capteur ;
- un organe d'affichage et/ou d'impression.

Avantageusement, en pratique :
- le rayon lumineux stabilisé est un rayon laser ;
- les trois capteurs sont constitués par des photodiodes ;
- le système à pression différentielle est constitué par un venturi ;
- l'échantillon à analyser s'écoule dans un capillaire au sein de la cellule d'analyse.

La manière dont l'invention peut être réalisée et les avantages qui en découlent ressortiront mieux de l'exemple de réalisation qui suit, donné à titre indicatif et non limitatif à l'appui des figures annexées, dans lesquelles :

La figure 1 représente une vue schématique du système d'écoulement de l'échantillon en vue de son analyse du dispositif conforme à l'invention.

Les figures 2 et 3 représentent des courbes établissant le nombre d'entités, à savoir débrits cellulaires et cellules entières, en fonction de la quantité d'ADN mesurée.

Conformément à la présente invention, l'échantillon de lait devant être analysé est tout d'abord soumis à un traitement préalable. En effet, on laisse incuber une quantité exactement mesurée de l'échantillon, et ce environ une vingtaine de minutes dans un milieu hypotonique, tel que du citrate de sodium 0,1% , en présence d'un agent tensio-actif (telle que du Triton (Marque Déposée)). La concentration de ce dernier est de l'ordre de 0,1 % en volume. On inclut également dans ce mélange réactionnel un colorant fluorescent dont la caractéristique essentielle est de se fixer spécifiquement sur l'Acide Desoxyribo Nucléïque (ADN) contenu dans le noyau des cellules et ce, de manière stoechiométrique. On choisit pour cela de l'iodure de propidium connu pour cette application. Afin d'être sûr que la totalité de l'ADN soit coloré, on ajoute l'iodure de propidium en excès.

Afin de ne pas exciter les molécules d'iodure de propidium, on réalise cette incubation à l'abri de la lumière.

L'agent tensio-actif permet une lésion des membranes cellulaires permettant une pénétration de l'iodure de propidium au niveau de l'ADN nucléaire.

De manière connue, toutes les cellules non tumorales d'une même espèce contiennent la même quantité d'ADN. De la sorte, la quantité de fluorescence mesurée ultérieurement constitue une donnée absolue quant à la quantité de cellules analysées.

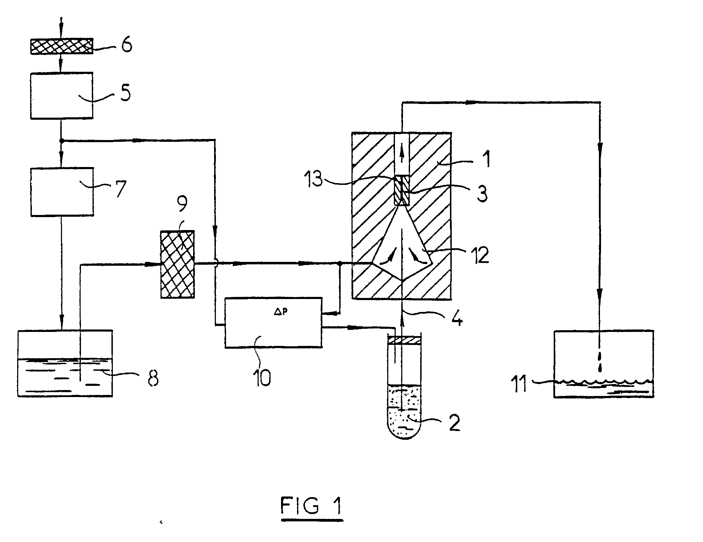

Afin d'assurer une mesure analytique de l'échantillon de lait à analyser, on a recours à un système de venturi à pression différentielle en liaison avec un capillaire permettant la création d'un courant laminaire de dimensions très réduites dudit échantillon, destiné à être placé, sous la forme d'un flux monocellulaire, sur le chemin optique du dispositif de mesure décrit ultérieurement. Pour ce faire, on fait appel au dispositif représenté au sein de la figure 1. Ce dispositif comprend une unité d'analyse dont l'élément essentiel est l'organe d'écoulement de l'échantillon. Ce dernier comprend fondamentalement une cellule de lecture réalisée en quartz apte à permettre la transmission d'un faisceau lumineux, notamment d'un faisceau laser. Les parois de cette cellule de lecture sont avantageusement planes et parallèles, afin de diminuer les éventuels phénomènes de reflexion partielle ou totale.

Une pompe à air (5), comprimant l'air extérieur préalablement filtré (6), et munie d'un régulateur de pression (7) permet d'envoyer le fluide porteur depuis son réservoir (8) jusqu'à la cellule de lecture (3), le dit fluide porteur, en l'occurence de l'eau permutée étant également filtré (9), notamment avec un filtre stérilisant, avant son introduction dans la chambre (12) du venturi. En parallèle, une boucle de contrôle fermée (10), régule la pression différentielle permettant à l'échantillon de lait (2) de passer par l'intermédiaire d'un tube capillaire (4) dans la cellule de lecture (3). Les parois de la chambre (12) du venturi convergent au niveau de la dite cellule de lecture (3), afin de former à ce niveau un capillaire (13). C'est dans ce capillaire (13) qu'est concentré le spot du faisceau lumineux utilisé pour le système de mesure et décrit ultérieurement.

Pour compléter l'unité de traitement (1) ainsi définie, on munit l'ensemble d'un réservoir (11) recueillant l'échantillon et le fluide support après analyse.

Le système de mesure conforme à l'invention comprend essentiellement un laser à Argon de 15 milliwatts, produisant un faisceau stabilisé de longueur d'onde bien déterminée égale à 488 nm. Le faisceau Laser ainsi produit est soigneusement collimaté par tout organe approprié et focalisé au niveau du capillaire (13) de la cellule de lecture (3). La longueur d'onde du faisceau laser produit tombe avantageusement au voisinage du maximum d'absorption de l'iodure de propidium, utilisé pour colorer l'ADN cellulaire. Lorsque ce dernier est excité, il réemet une radiation dite de fluorescence de longueur d'onde bien connue et égale à 620 nm. Cette radiation de fluorescence est détectée au moyen d'un capteur constitué essentiellement d'un photomultiplicateur, mesurant l'intensité du radiation émise selon un signal $I_1$, et de fait, la quantité d'ADN s'étant écoulé devant le faisceau laser. Cette dernière grandeur permet de remonter au nombre de cellules ayant transité par le capillaire (13), car comme déjà dit, la quantité d'ADN cellulaire est une constante constituant de la sorte un quantum de repère. Il est à noter qu'il existe toutefois un petit nombre ce cellules en phase de mitose. De fait, leur quantité d'ADN est double par rapport aux cellules en interphase.

Il va de soi que pour parvenir à une précision suffisante, le capillaire (13) doit avoir des dimensions suffisamment réduites afin de n'autoriser l'écoulement simultané que d'un nombre très limité de cellules, pour que la totalité des cellules émette une radiation de fluorescence via l'iodure de propidium.

L'information $I_1$ ainsi obtenue est en elle-même nécessaire et suffisante pour estimer la qualité du lait. Toutefois, afin de disposer d'éléments supplémentaires d'appréciation, on procède à d'autres mesures. Ainsi, une partie du faisceau laser est diffractée par les cellules selon une grande variété d'angles par rapport à l'axe optique défini par le faisceau lui-même. On place de fait à 90 degrés par rapport à cet axe optique un second capteur, constitué par une photodiode, afin de délivrer un signal $I_2$ utilisable. Ce signal $I_2$ est représentatif de la densité des cellules s'écoulant dans le capillaire (13).

On place aussi à environ 10 degrés de l'axe optique défini précédemment un troisième capteur, également constitué par un photomultiplicateur, et détectant une partie du faisceau laser diffracté par les cellules. Les informations détectées à ce niveau correspondent à une partie du faisceau laser ayant été diffractée lors de la traversée des cellules par ledit faisceau. De la sorte, l'information $I_3$ que ce capteur restitue est représentatif de la taille des dites cellules.

De la sorte, on dispose d'au moins trois informations $I_1$, $I_2$ et $I_3$ correspondant respectivement au nombre, à la taille et à la densité des cellules s'écoulant dans le capillaire (13).

Ces informations sont traitées au moyen d'un microprocesseur susceptible d'effectuer une selection parmi les informations transmises, en fonction de paramètres introduits dans le dit microprocesseur par l'utilisateur.

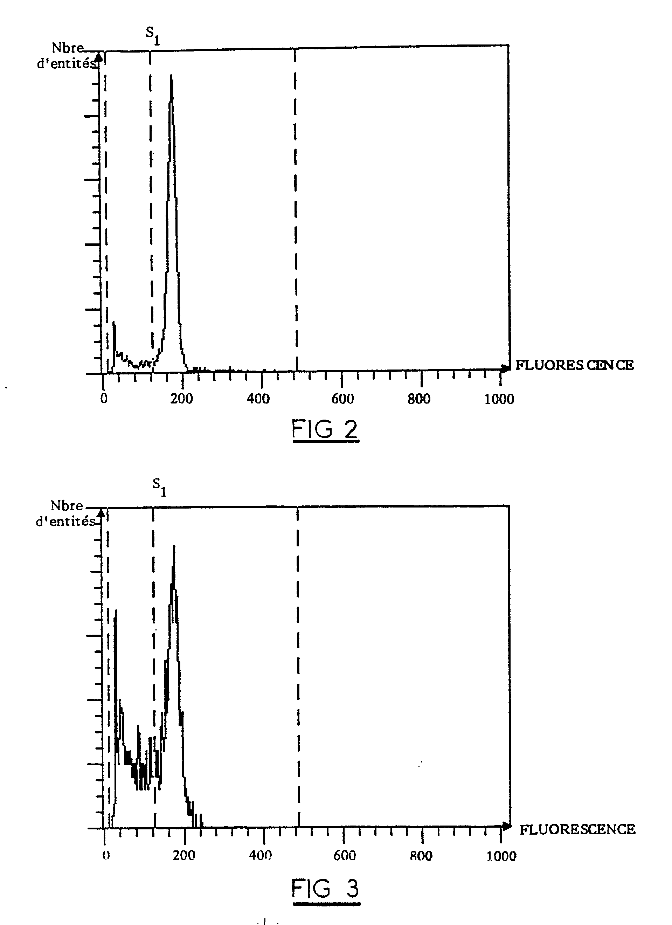

Il est par exemple nécessaire dans la détermination du nombre de cellules par unité de volume de lait, de s'affranchir des cellules non entières. Il suffit dans ce cas de fixer un seuil pour la fluorescence,en dessous duquel les entités détectées ne sont pas comptabilisées comme des cellules, mais comme des débris, d'origine cellulaire. Ce seuil est très facilement déterminé. Il correspond en effet, à la quantité de fluorescence équivalente, dans le cas du lait, donc des mammifères à la quantité d'ADN contenue dans le stock diploïde des 2n chromosomes. De fait, toutes les entités, dont l'intensité de fluorescence dépasse ce seuil sont des cellules entières. Dans les figures 2 et 3, on a représenté par $S_1$ ce seuil. On observe sur ces courbes, un pic très marqué, correspondant aux cellules entières en interphase. La détermination de la quantité d'ADN au dessus de ce seuil permet donc d'obtenir avec précision le nombre de cellules entières, aux cellules en cours de mitose près.

Comme déjà dit, toutes les entités dont le signal de fluorescence apparait en dessous du seuil $S_1$ sont des débris cellulaires.

De manière avantageuse, le rapport des débris cellulaires sur la quantité d'ADN totale et le rapport des cellules entières sur la quantité d'ADN totale sont déterminés, afin de donner avec une plus grande précision l'état pathologique de l'animal concerné.

De même, la différenciation entre particules cellulaires et non cellulaires (par exemple globules lipidiques, très nombreux dans le cas du lait) s'effectue, dans le cas de mesure de fluorescence directe, par la sélection d'une plage d'intensité correspondant à la quantité connue d'ADN cellulaire.

De la sorte, on obtient une valeur représentative de la qualité du lait analysé, sous la forme d'un nombre de cellules par unité de volume, voire d'un graphique matérialisant les différentes informations mesurées, tels que ceux représentés au sein des figures 2 et 3, et ce au moyen de tout organe de restitution sensorielle appropriée, tel que par exemple un écran et/ou une imprimante.

L'utilisateur est donc à même de déterminer rapidement la qualité de son lait. De fait, les litiges potentiels entre producteurs et laiteries, ou entre laiteries et consommateurs peuvent être largement diminués, la résultante générale conduisant à l'amélioration de la qualité du lait.

Dans une mise en oeuvre avantageuse de l'invention, il est possible de procéder à une coloration multiple, en faisant appel à des colorants bien spécifiques, et ce, en vue d'obtenir la population de ou des différents types cellulaires contenus dans le lait, à savoir, par exemple, les polynucléaires, les lymphocytes, les macrophages ou monocytes et les cellules épithéliales.

Pour ce faire, on a recours à une réaction enzymatique propre à chacune desdites espèces. En d'autres termes, on fait réagir, lors de l'étape de coloration, un substrat d'une enzyme spécifique contenue dans l'espèce dont on désire mesurer la population.

Par exemple, lorsque l'on désire connaître, outre la population totale en cellules d'un échantillon de lait, la population particulière en polynucléaires, on adjoint à l'iodure de propidium du phosphate de méthyl fluorescéïne, ce dernier réagissant spécifiquement avec lesdits polynucléaires.

Le processus de l'analyse est alors rigoureusement le même que celui précédemment décrit, à savoir phase d'excitation à 488 nm, seule la phase de lecture s'effectuant à une longueur d'onde de 520 nm.

De la sorte, les résultats obtenus permettent de préciser la quantité relative de polynucléaires, donnée particulièrement intéressante pour établir un diagnostic, notamment, lorsque le seuil fixé à 500 000 cellules par millilitre n'est pas atteint.

Ainsi, le procédé conforme à l'invention permet-il d'une part une numérisation des différents constituants cellulaires du lait par des colorants spécifiques tel l'Iodure de propidium et tous ses équivalents, et d'autre part, une caractérisation des cellules comptées au moyen d'un marquage enzymatique spécifique d'un type cellulaire avec des substrats fluorescents.

Enfin, l'utilisation du cadrage des plages cellulaires présentant des différences de taille et/ou de nature permet d'améliorer sensiblement la précision du diagnostic.

## Revendications

1/ Procédé pour identifier et numériser les cellules contenues dans du lait **caractérisé** en ce qu'il consiste :

- à prélever une quantité déterminée d'un échantillon d'un lait à analyser ;
- à colorer les cellules présentes dans cet échantillon par un colorant fluorescent spécifique de l'ADN (Acide desoxyribonucléïque) contenu dans le noyau desdites cellules ;
- à faire défiler cet échantillon coloré dans un tube capillaire (13) sur lequel est dirigé un faisceau lumineux stabilisé ;
- à recueillir sur un capteur une partie au moins du rayon lumineux transmis à travers le flux de l'échantillon s'écoulant dans le tube capillaire (13) pour donner une première information $I_1$ significative de la quantité d'ADN, donc du nombre de cellules qui défilent;
- à transmettre ladite information $I_1$ à une unité de traitement ;
- et enfin, à restituer au moins une valeur représentative de la qualité du lait analysé.

2/ Procédé selon la revendication 1, caractérisé :
- en ce que, lors de la phase de coloration, on ajoute en outre un autre colorant fluorescent, apte à induire une réaction enzymatique spécifique d'une famille des constituants contenus dans lesdites cellules ;
- et, en ce que, l'on recueille sur un capteur, partie du rayon lumineux transmis à travers le flux de l'échantillon s'écoulant dans le tube capillaire (13) pour donner une information, représentative de la quantité de la dite famille de constituants cellulaire .

3/ Procédé selon la revendication 1, caractérisé en ce que, simultanément, on recueille sur des capteurs, respectivement :
- une partie du rayon lumineux diffracté à 90° de l'axe optique défini par le faisceau lumineux initial pour donner une seconde information $I_2$, significative de la densité desdites cellules ;
- au moins une partie du rayon lumineux légèrement diffracté au voisinage de l'axe optique, pour donner une troisième information $I_3$, significative de la taille desdites cellules ;
les dites informations $I_2$ et $I_3$ étant alors transmises à la dite unité de traitement puis restituées sous forme de valeur(s) représentative(s) de la qualité du lait analysé.

4/ Procédé selon l'une des revendications 1 à 3, caractérisé en ce que simultanément à la coloration, on ajoute au lait un milieu hypotonique , destiné à lyser la membrane cellulaire, et un détergent tensio-actif destiné à perméabiliser la membrane nucléaire afin de permettre la pénétration du colorant fluorescent destiné à venir se fixer sur l'ADN.

5/ Procédé selon la revendication 4, caractérisé en ce que l'agent tensio-actif est introduit dans le milieu à raison de 0,1 % en volume, et l'agent hypotonique est du citrate de sodium à raison de 0,1 % en rapport Poids/Volume.

6/ Procédé selon l'une des revendications 1 à 5, caractérisé en ce que le colorant fluorescent spécifique de l'ADN est de l'iodure de propidium, introduit en excès par rapport à la quantité d'ADN .

7/ Procédé selon l'une des revendications 1 à 6, caractérisé en ce qu'on laisse incuber le milieu réactionnel à température ambiante et à l'abri de la lumière pendant au moins vingt minutes .

8/ Procédé selon l'une des revendications 1 à 7, caractérisé en ce qu'on fait écouler le milieu réactionnel au moyen d'un venturi (5,7,8,10,12) à pression différentielle.

9/ Procédé selon l'une des revendications 1 à 8, caractérisé en ce que le faisceau lumineux stabilisé est un faisceau laser.

10/ Dispositif pour la mise en oeuvre du procédé selon l'une des revendications 1 à 9, caractérisé en ce qu'il comprend :
- un laser stabilisé ;
- une unité d'analyse (1) constituée par :
. une cellule de lecture (3) réalisée en quartz, située sur le trajet optique du faisceau laser ;
. un système à pression différentielle destiné à permettre le passage dans ladite cellule de lecture (3) de l'échantillon de lait à analyser (2) ;
. un réservoir (11) destiné à recueillir l'échantillon après analyse ;
- un laser stabilisé ;
- trois capteurs optiques, dont deux sont munis d'un photomultiplicateur ;
- un microprocesseur apte à assurer le traitement des informations issues dudit capteur ;
- un organe d'affichage et/ou d'impressions.

11/ Dispositif selon la revendication 10, caractérisé :
- en ce que les capteurs sont constitués par des photodiodes ;
- en ce que l'échantillon à analyser (2) s'écoule dans un capillaire (13) au sein de la cellule de lecture (3) ;
- et, en ce que le système à pression différentielle est constitué par un venturi.

FIG 1

FIG 2

FIG 3

# RAPPORT DE RECHERCHE EUROPEENNE

Office européen des brevets

Numero de la demande: EP 90 42 0212

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | BIOLOGICAL ABSTRACTS, vol. 82, no. 12, 1986, pages AB-458, résumé no. 112110, Philadelphia, PA, US; M. HAGELTORN et al.: "Flow cytofluorometric characterization of bovine blood and milk leukocytes", & AM. J. VET. RES., 47(9): 2012-2016, 1986 * Résumé * | 1 | C 12 Q 1/04<br>G 01 N 33/50<br>G 01 N 33/04<br>G 01 N 1/30 |
| A | IDEM | 4 | |
| Y | US-A-3 679 365 (K.B. WRIGHTMAN et al.) * Colonnes 1-2 * | 1,6 | |
| Y | CLINICAL CHEMISTRY, vol. 29, no. 2, février 1983, pages 290-296, Washington, US; D.C. SHELLY et al.: "Characterization of bacteria by mixed-dye fluorometry" * En entier * | 1,6 | |
| A | EP-A-0 268 766 (TOA MEDICAL ELECTRONICS CO., LTD) * Page 6, ligne 12 - page 8, ligne 3; revendications * | 1,3,4,9,10 | DOMAINES TECHNIQUES RECHERCHES (Int. Cl.5)<br><br>C 12 Q<br>G 01 N |
| A | BIOLOGICAL ABSTRACTS, vol. 86, no. 10, 1988, page AB-1153, résumé no. 108307, Philadelphia, PA, US; D. REDELMAN et al.: "Identification of inflammatory cells in bovine milk by flow cytometry", & CYTOMETRY 9(5): 463-468, 1988 * Résumé * | 1,3 | |

Le présent rapport a été établi pour toutes les revendications

| Lieu de la recherche | Date d'achèvement de la recherche | Examinateur |
|---|---|---|
| LA HAYE | 11-06-1990 | HITCHEN C.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)